# EUROPEAN PATENT SPECIFICATION

(11) **EP 4 608 314 B1**
(45) Date of publication and mention of the grant of the patent: **25.11.2026**
(21) Application number: 23806113.9
(22) Date of filing: 24.10.2023
(51) Int. Cl.: A61B 18/22

(54) **ENDOSCOPIC SURGICAL SYSTEMS**
ENDOSKOPISCHE CHIRURGISCHE SYSTEME
SYSTÈMES CHIRURGICAUX ENDOSCOPIQUES

(30) Priority: 27.10.2022 US 202263381222 P
(43) Date of publication of application: 03.09.2025
(73) Proprietor: Boston Scientific Scimed Inc., Maple Grove, Minnesota 55311 (US)
(72) Inventor: CARLSON, Steven T., St. Paul, Minnesota 55118 (US); RAUNIYAR, Niraj Prasad, Plymouth, Minnesota 55446 (US); JOHNSON-KIDD, Tiana, Minneapolis, Minnesota 55408 (US)
(74) Representative: Pfenning, Meinig & Partner mbB
(86) International application number: PCT/US2023/035772
(87) International publication number: WO 2024/091482

(56) References cited:
- WO-A1-96/11638
- US-A- 6 024 751
- US-A1- 2017 265 879

## Description

### TECHNICAL FIELD

Various aspects of this disclosure relate generally to systems, devices, and methods useful in medical procedures. More specifically, this disclosure relates to systems, devices, and methods for accessing, breaking up, removing, or otherwise treating hardened masses, tissue, or other objects from a body lumen.

### BACKGROUND

Lithotripsy is a medical procedure involving the physical disruption of a hardened mass or object within a body cavity, such as kidney stones, gallstones, pancreatic stones, or the like. In such procedures, energy is applied to the hardened mass or object. Different energy sources may be used, such as electric, hydraulic, laser, mechanical, ultrasound, or the like. In laser lithotripsy, pulsed light energy from an energy delivery device may be converted into a mechanical energy in the form of a cavitation bubble associated with the occurrence of a shockwave. This mechanical energy may facilitate disruption and/or breaking up of the hardened mass or object.

Many lithotripsy procedures generate particles, dust, or pollution within the body cavity as the hardened mass or object is disrupted and broken-up. For stone-like masses, these particles may be referred to as "stone dust." In laser lithography procedures, this stone dust may hinder visualization of the mass or object, which may prevent the physician from locating the mass or object and delivering the laser energy in the most efficient manner. Fluid may be injected into the body cavity so that a portion of the stone dust will naturally drain out of the body cavity with the fluid. However, stone dust or other particles may not drain out of certain body cavities or portions of a body cavity, for example, a lower pole of a kidney. Suction may be delivered to the body cavity to help remove the stone dust, but applying suction may require either removing the energy delivery device or inserting an additional medical device into the body cavity, which may prolong the procedure or expose the subject to contamination or other risks. Furthermore, the suction device may be susceptible to clogs or reduced fluid flow if stone dust or other particles accumulate within the suction lumen or if larger stones or particles enter the suction lumen. The aforementioned risks may increase the cost, time, and necessary personnel for a medical procedure, further complicating and prolonging the procedure, and exposing the subject to greater risk.

The systems, devices, and methods of this disclosure may rectify some of the deficiencies described above, and/or address other aspects of the prior art.

Document US 6 024 751 A describes a method and apparatus for transurethral resection of the prostate, which includes inserting a transurethral incisional device through the patient's urethra, incising off at least one piece of targeted prostatic tissue using the incisional device, inserting a morcellation probe through the patient's urethra, morcellating the excised piece of targeted prostatic tissue with the morcellation probe, and aspirating the morcellated prostatic tissue through the morcellation probe and out of the patient. The morcellation probe includes an elongated inner probe tube that defines an aspiration channel therein. The inner probe tube is slidably disposed inside an outer probe tube. The outer probe tube has a first aperture with a first cutting edge for cutting tissue. The inner probe tube has a second aperture or a spiral shaped groove defining a second cutting edge that moves relative to the first cutting edge to cut tissue drawn through the first aperture. The second aperture or groove are in communication with the aspiration channel. A tissue manipulation port can be formed in either the outer or inner probe tubes to overlap with the inner or outer probe tube apertures or the groove to provide continuous aspiration adjacent the first aperture if/when the first aperture is sealed by the inner probe tube.

Document US 2017/265879 A1 describes an integrated clot evacuation device having visualization for use in neurosurgical applications, particularly for the evacuation of clots formed as a result of intracranial hemorrhage (ICH). The device may further include an integrated camera and light for visualizing the interior of the brain and the clot itself. Further, the device is configured to evacuate clots through aspiration and irrigation.

Document WO 96/11638 A1 describes a tissue resection device comprising a handle housing having a fluid infusion lumen. A shaft is reciprocally mounted to the housing, the shaft having an aperture adjacent to a distal end and a fluid and tissue aspiration lumen extending from the aperture to a proximal end of the shaft. A cutting member is disposed adjacent to the aperture to sever tissue as the shaft is reciprocated, and an imaging mechanism on the housing is oriented toward the cutting member thereby allowing the attending surgeon to optically direct the removal of body cavity tissue. A chopping mechanism is disposed within the lumen of the shaft to reduce the size of tissues passing through the lumen.

### SUMMARY

The invention is set out in the appended set of claims. Examples of this disclosure relate to, among other things, medical systems, devices, and methods. Each of the examples disclosed herein may include one or more of the features described in connection with any of the other disclosed examples. Any example, which is in contradiction to the subject-matter of claim 1, is not part of the invention.

In accordance with the invention, a medical system includes a sheath with at least one lumen, an irrigation device, and a medical device. The at least one lumen is configured to be coupled to a suction source. The irrigation device includes at least one irrigation lumen configured to be coupled to an irrigation source. The medical device includes a handle and a shaft. The shaft includes a working channel with a distal opening at a distal end of the shaft. The distal end of the shaft further includes one or more illumination devices and one or more visualization devices. The medical device is configured to be positioned within the sheath during delivery of the sheath to a treatment site and during an application of energy to one or more objects to break up the one or more objects to form one or more particles or dust. The irrigation device is configured to be positioned within the sheath and deliver irrigation fluid while suction is applied to the treatment site through the sheath during a removal of one or more particles or dust from the treatment site. A distal portion of the shaft of the medical device includes an articulation section. The handle of the medical device includes a deflection lever that is configured to be manipulated to control a position of the articulation section of the shaft.

The medical system may include one or more of the following features. The medical system may further include a laser fiber or another energy delivered device, optionally wherein the laser fiber or the another energy delivery device is delivered through the working channel of the medical device. The laser fiber or the another energy delivery device may be coupled to or integrated within the shaft of the medical device. The irrigation device may include a closed distal end and a plurality of holes arranged on a distal portion of the irrigation device. The plurality of holes may be arranged in at least one row of holes, e.g., at least two rows of holes arranged on opposing sides of the distal portion of the irrigation device. The plurality of holes may be formed via laser cutting.

The irrigation device may include a crescent cross-sectional shape with an open distal end. The irrigation device may include at least one helical section. The sheath may include an additional lumen. The additional lumen may be at least partially separated from the lumen of the sheath. The additional lumen may be configured to movably receive the irrigation device while the shaft of the medical device is positioned within the lumen of the sheath. The additional lumen may include a sliver shape formed by a first wall with a first radius of curvature and a second wall with a second radius of curvature that is greater than the first radius of curvature. The handle may be coupled to an umbilicus configured to couple the handle to one or more of an irrigation source, a suction source, a display, or a power source. The treatment site may be a kidney, and the one or more objects may be kidney stones.

In another aspect, a medical system may include a sheath with at least one lumen, an irrigation device, and a medical device. The at least one lumen may be configured to be coupled to a suction source. The irrigation device may include at least one irrigation lumen configured to be coupled to an irrigation source. The irrigation device may include a shape that is substantially complementary to an interior surface of the at least one lumen of the sheath in order to retain or maintain a position of the irrigation device within the sheath with at least a portion of the irrigation device abutting an inner wall of the at least one lumen of the sheath. The medical device may include a handle and a shaft. The handle may include a deflection lever. The shaft may include a working channel with a distal opening at a distal end of the shaft. A portion of the shaft may include an articulation section controlled by the deflection lever. The medical device may be configured to be positioned within the sheath during delivery of the sheath to a treatment site and during an application of energy to one or more objects to break up the one or more objects to form one or more particles or dust. The irrigation device may be configured to be positioned within the sheath and deliver irrigation fluid while suction is applied to the treatment site through the sheath during a removal of one or more particles or dust from the treatment site.

The medical system may include one or more of the following features. The irrigation device may include a crescent cross-sectional shape with an open distal end. The irrigation device may include at least one helical section.

In yet another aspect, a medical system may include a sheath, a steering tube including a lumen and configured to be movably positioned within the sheath, and an irrigation device. The sheath may include a cylindrical shape. The steering tube may be formed of a shape-memory material. A distal portion of the steering tube may include a radius of curvature of approximately 90 degrees when in an unconstrained configuration. The irrigation device may include a lumen. The irrigation device may be configured to be movably positioned within the steering tube. The irrigation device may be formed of a shape-memory material. A distal portion of the irrigation device may include a radius of curvature of approximately 180 degrees when in an unconstrained configuration.

The medical system may include one or more of the following features. The irrigation device may be less rigid than the steering tube. The irrigation device may be less rigid than the sheath. The sheath may be configured to be delivered to a ureter. The steering tube and the irrigation device may be configured to be delivered into a kidney to deliver irrigation and/or suction to one or more kidney stones or one or more calyxes.

Both the foregoing general description and the following detailed description are exemplary and explanatory only and are not restrictive of the features, as claimed. As used herein, the terms "comprises," "comprising," "having," "including," or other variations thereof, are intended to cover a non-exclusive inclusion such that a process, method, article, or apparatus that comprises a list of elements does not include only those elements, but may include other elements not expressly listed or inherent to such a process, method, article, or apparatus. Additionally, the term "exemplary" is used herein in the sense of "example," rather than "ideal." As used herein, the terms "about," "substantially," "generally," and "approximately," indicate a range of values within +/-10% of a stated value.

### BRIEF DESCRIPTION OF THE DRAWINGS

The accompanying drawings, which are incorporated in and constitute a part of this specification, illustrate various exemplary embodiments and together with the description, serve to explain the principles of the disclosure.
FIG. 1 illustrates a medical system, according to aspects of this disclosure.
FIGs. 2A-2C illustrate perspective views of distal portions of the medical system of FIG. 1 in various configurations, according to aspects of this disclosure.
FIGs. 3A and 3B illustrate perspective views of distal portions of another medical system in various configurations, according to aspects of this disclosure.
FIGs. 4A-4C illustrate perspective views of distal portions of a further medical system in various configurations, according to aspects of this disclosure.
FIGs. 5A-5C illustrate perspective views of distal portions of yet another medical system in various configurations, according to aspects of this disclosure.
FIGs. 6A and 6B illustrate perspective views of distal portions of a further medical system in various configurations, according to aspects of this disclosure.
FIG. 7A illustrates distal portions of another medical system, and FIGs. 7B-7D illustrate distal portions of the another medical system of FIG. 7A in a kidney of a subject in various configurations, according to aspects of this disclosure.

### DETAILED DESCRIPTION

Examples of this disclosure include systems, devices, and methods to facilitate and improve the efficacy, efficiency, and safety of medical procedures to break up and remove hardened masses or objects. For example, aspects of this disclosure may provide an operator (e.g., a physician, medical technician, or other medical service provider) with the ability to more easily apply energy (e.g., laser energy) from an energy source to a stone (e.g., a kidney stone), deliver irrigation fluid, and/or apply suction to remove dust or particles. Aspects of this disclosure may allow an operator to deliver energy, deliver irrigation fluid, and/or apply suction within a body cavity to remove small stones or stone dust, and breakup larger stones or objects to be removed without the need to remove medical devices from the body cavity. Additionally, aspects of this disclosure may be used in performing an endoscopic, hysteroscopic, or ureteroscopic procedure.

Reference will now be made in detail to examples of this disclosure described above and illustrated in the accompanying drawings. Wherever possible, the same reference numbers will be used throughout the drawings to refer to the same or like parts.

The terms "proximal" and "distal" (e.g., arrow "P" and arrow "D" in various figures) are used herein to refer to the relative positions of the components of an exemplary medical device or an insertion device. When used herein, "proximal" refers to a position relatively closer to the exterior of the body or closer to an operator using the medical device or insertion device. In contrast, "distal" refers to a position relatively farther away from the operator using the medical device or insertion device, or closer to the interior of the body.

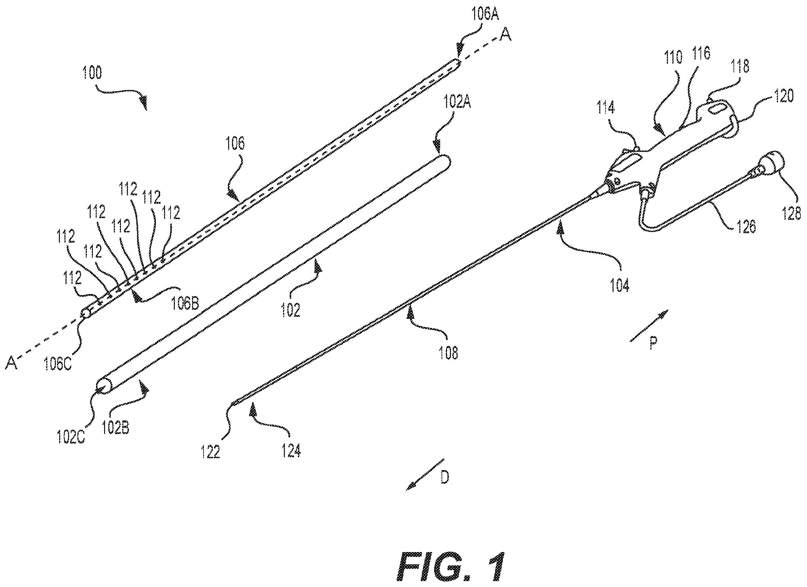

FIG. 1 illustrates a medical system 100 that includes an insertion device or sheath 102, a medical device 104, and an irrigation device 106. Sheath 102 may be generally tubular, for example, including a proximal end 102A, a distal end 102B, and a lumen 102C extending from proximal end 102A to distal end 102B. Medical device 104 includes a shaft 108 (e.g., a delivery shaft) and a handle 110. Shaft 108 extends from a distal end of handle 110. Irrigation device 106 may be generally tubular, for example, including a proximal end portion 106A and a distal end portion 106B. Irrigation device 106 may include one or more holes 112, for example, on distal end portion 106B, for example, proximal of distalmost end 106C. Distalmost end 106C may be closed. Irrigation device 106 include an interior lumen (not shown), for example, extending from proximal end portion 106A to a closed distalmost end 106C, such that irrigation fluid may be delivered through the interior lumen and out of holes 112. Portions of one or more of medical device 104 (i.e., shaft 108) and irrigation device 106 may be delivered through lumen 102C of sheath 102 to deliver fluid and/or apply suction to help remove one or more objects from a subject. Moreover, as discussed below, one or more portions of medical device 104 may be used to deliver energy to one or more objects within the subject, for example, to break up the one or more objects to make removal easier.

Sheath 102 may be an insertion sheath. Sheath 102 may be formed of any biocompatible material. Sheath 102 may be formed of a flexible material, for example, such that sheath 102 may be manipulated and/or delivered to one or more lumens within the subject, for example, to a kidney (e.g., into one or more calyxes). Although not shown, proximal end 102A of sheath 102 may be coupled to one or more of a fluid source (e.g., a fluid pump), a suction source (e.g., a suction pump or wallmounted suction sources), etc. Moreover, although not shown, proximal end 102A of sheath 102 may include one or more valves, ports, etc.

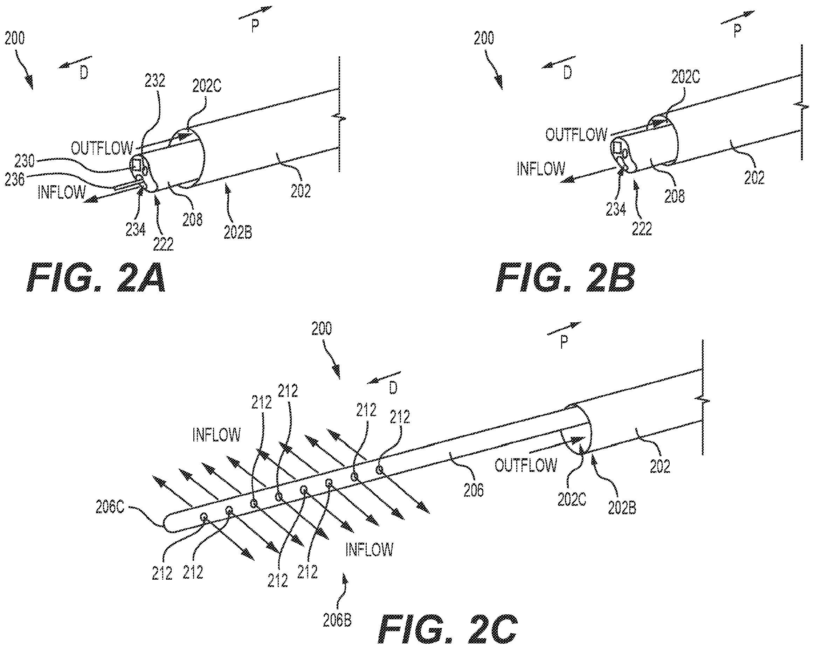

As mentioned, medical device 104 includes shaft 108 and handle 110. Handle 110 may include one or more ports 114, one or more valves 116, and/or one or more control or actuation elements 118. Port(s) 114 may be positioned on a distal portion of handle 110. Port(s) 114 may be fluidly connected to a working channel (not shown) in shaft 108 with a distal opening 234 (FIG. 2A) at a distal tip 122 of shaft 108. Port(s) 114 may connect to the working channel through at least one internal lumen (not shown) in handle 110. Port(s) 114 may include a T-connector, a Y-connector, or another appropriate connector. Port(s) 114 may be threaded, may be a Luer component, and/or may include one or more internal flexible seals. Port(s) 114 may be configured to receive on or more instruments (e.g., graspers, guide wires, needles, cautery devices, baskets, etc.) One or more valves 116 may be configured to be actuated to control the delivery of irrigation fluid and/or application of suction, for example, through the working channel of shaft 108. Control or actuation elements 118 may be configured to control or actuate one or more aspects of medical device 104, for example, to capture one or more images with one or more imaging devices, illuminate the treatment site by activating one or more illumination devices, or otherwise actuate or control one or more other aspects of medical device 104.

Medical device 104 also includes one or more deflection levers 120, for example, on a proximal portion of handle 110. Deflection lever(s) 120 may be manipulated to deflect or otherwise control the position of one or more portions of shaft 108, for example, of a deflection or articulation section 124 of shaft 108. Deflection lever(s) 120 and deflection or articulation section 124 may help to allow for shaft 108 to be deflected in up, down, left, and/or right directions. In one example, articulation section 124 may provide for full retroflexion (e.g., rotation of distal tip 122 through an arc of 180 degrees) or only partial retroflexion (e.g., rotation of distal tip 122 through an arc of less than 180 degrees).

Moreover, medical device 104 may be coupled to an umbilicus 126. Umbilicus 126 may extend from handle 110 to one or more auxiliary devices. Umbilicus 126 may include an umbilicus coupling 128, for example, to couple umbilicus 126 and medical device 104 to one or more auxiliary devices. The one or more auxiliary devices may include a controller or control system, an imaging system, a power supply, a display, a fluid supply, a suction/vacuum source, etc.

In some aspects, medical device 104 may be an ureteroscope (e.g., LithoVue^{™} Single-Use Digital Flexible Ureteroscope by Boston Scientific Corp.), an endoscope, a hysteroscope, a bronchoscope, a cystoscope, a duodenoscope, a colonoscope, a gastroscope, an endoscopic ultrasonography ("EUS") scope, a laparoscope, an arthroscope, an aspiration scopes, or any similar device. Medical device 104 may be for single-use and be disposable, or medical device 104 may be reusable.

Irrigation device 106 may be generally tubular. Additionally, irrigation device 106, for example, distal end portion 106B may include a plurality of holes 112. Holes 112 may be arranged linearly (e.g., parallel to a longitudinal axis A of irrigation device 106) along distal end portion 106B. In some aspects, irrigation device 106 may include two rows of holes 112, for example, on opposing sides of irrigation device 106 (e.g., spaced approximately 180 degrees apart around a circumference of irrigation device 106). Alternatively, irrigation device 106 may include one row of holes 112, three rows of holes 112, four rows of holes 112, five rows of holes 112, six rows of holes 112, etc. In some examples, each row of holes 112 may be evenly spaced circumferentially about irrigation device 106. Each row of holes 112 may include two, three, four, five, six, seven, eight, ten, twenty, fifty, or more holes 112. Holes 112 may be formed via a laser cutting of various portions of distal end portion 106B.

In these aspects, sheath 102, medical device 104 (e.g., shaft 108), and/or irrigation device 106 may be appropriate lengths and/or flexibilities, for example, to be delivered to one or more lumens (e.g., a kidney) of the subject. In these aspects, proximal ends of each of sheath 102, medical device 104 (e.g., handle 110), and/or irrigation device may remain proximal to the subject. Moreover, portions of one or more of irrigation device 106 and shaft 108 may be delivered through lumen 102C of sheath 102.

FIGs. 2A-2C illustrate a medical system 200 (e.g., a distal portion of medical system 200) in various configurations. Where possible, corresponding reference numbers to medical system 100 (i.e., reference numbers including the same second and third digits) are used to refer to corresponding structure. As shown in FIG. 2A, shaft 208 may be positioned within (e.g., radially within) sheath 202. Distal tip 222 of shaft 208 may include one or more illumination devices 230 (e.g., light emitting diodes, optical fibers, etc.) and one or more imaging devices 232 (e.g., cameras, imagers, etc.). Illumination device(s) 230 and imaging device(s) 232 may be coupled to one or more power sources, controllers, displays, etc. via one or more wires, cables, etc. extending proximally through shaft 108, handle 110, and/or umbilicus 126 (FIG. 1). Moreover, distal tip 222 includes a distal opening 234 of the working channel extending through shaft 208.

Shaft 208 may be movable relative to sheath 202. In some aspects, shaft 208 may be positioned within sheath 202, and shaft 208 may be used to deflect or otherwise position shaft 208 and sheath 202 through one or more lumens and to the treatment site. In this aspect, sheath 202 may be flexible, such that sheath 202 moves with shaft 208 when shaft 208 is deflected. For example, one or more of illumination devices 230 and/or imaging devices 232 may be activated to provide visualization and help the user deliver sheath 202 and shaft 208 to the treatment site. Furthermore, deflection lever 120 (FIG. 1) may be manipulated to help deflect one or more portions of shaft 208, and also manipulate sheath 202.

As shown in FIG. 2A, distal tip 222 of shaft 208 may be extended distally of distal end 202B of sheath 202. Moreover, a laser fiber 236 or other energy delivery device/element may be delivered to the treatment site via shaft 208, for example, through distal opening 234 of the working channel. Alternatively, laser fiber 236 or other energy delivery device/element may be integrated into shaft 208, for example, built into shaft 208 or otherwise positioned on distal tip 222. Although not shown and in yet another alternative, laser fiber 236 may be delivered through lumen 202C of sheath 202, for example, radially outside of or otherwise adjacent to shaft 208. In these aspects, laser fiber 236 may be activated (e.g., via one or more control or actuation elements 118 on handle 110, shown in FIG. 1) to direct laser energy (or other forms of energy) toward one or more objects at the treatment site. In some examples, the one or more objects may be stones positioned in one or more portions of the subject's kidney. In these aspects, the delivery of the energy may help to break up the one or more objects at the treatment site. In some aspects, breaking up the one or more objects may form small particles or dust from the one or more objects.

As shown in FIG. 2A, fluid (e.g., irrigation fluid) may be delivered to the treatment site via the working channel, such that the fluid inflows to the treatment site out of distal opening 234 of the working channel, for example, at least partially around laser fiber 236. The fluid may help to remove particles or dust from the area adjacent to distal tip 222. Clearing the area adjacent to distal tip 222 (e.g., a field of view of imaging device(s) 232) may help clear the area around one or more illumination devices 230 and the one or more imaging devices 232, which may help the user visualize the one or more objects or other aspects of the treatment site.

Furthermore, suction may be applied to the treatment site through sheath 202, for example, to create an outflow, as shown in FIG. 2A. The suction may help to remove small particles or dust, for example, formed when the energy delivered by laser fiber 236 breaks up the one or more objects at the treatment site. Furthermore, the laser fiber 236, the delivery of irrigation fluid to create the inflow, and/or the application of suction to create the outflow may be alternatingly actuated, contemporaneously actuated, or otherwise controlled to help break up material (e.g., stones, particles, tissue, dust, etc.), clear the area adjacent to distal tip 222, and/or remove the material from the treatment site.

FIG. 2B illustrates the distal end of medical system 200 in another configuration. As shown in FIG. 2B, laser fiber 236 may be removed or at least partially retracted proximally within the working channel of shaft 208. In this aspect, a greater volume of irrigation fluid may be delivered through the working channel of shaft 208 and out of distal opening 234. The inflow of irrigation fluid may help to clear the area around distal tip 222 of shaft 208. The inflow of irrigation fluid may help to flush one or more portions of the treatment site (e.g., one or more calyxes of a kidney). As discussed above, suction may be applied through sheath 202, such that particles and/or dust may be removed proximally from the treatment site via lumen 202C of sheath 202 and around shaft 208.

FIG. 2C illustrates the distal end of medical system 200 in yet another configuration. As shown, shaft 208 may be removed proximally from sheath 202. Additionally, irrigation device 206 may be delivered through lumen 202C of sheath 202. Distal portion 206B of irrigation device 206 may be extended distally beyond distal end 202B of sheath 202. Irrigation fluid may be delivered to the treatment site through irrigation device 206, for example, out of the plurality of holes 212. As mentioned, distal portion 206B of irrigation device 206 may include two rows of holes 212, for example, positioned on opposing sides of irrigation device 206 to deliver irrigation fluid out of opposing sides of irrigation device 206. Delivering irrigation fluid via irrigation device 206 may help to disperse irrigation fluid at multiple points of irrigation device 206. Moreover, irrigation device 206 may be extended and/or retracted from sheath 202, for example, such that distal end 206C may be positioned at various positions relative to the treatment site (e.g., into one or more calyxes of a kidney). In some aspects, irrigation device 206 may be directly or indirectly deflectable, for example, to further control the position of distal end 206C. In these aspects, irrigation device 206 may help to flush one or more portions of the treatment site. In some aspects, as shown in FIG. 2C, distal end 206C may be rounded, curved, or otherwise at least partially atraumatic, to help prevent damage or injury at the treatment site as irrigation device 206 is moved relative to the treatment site, for example, as distal end 106C may abut or otherwise contact portions of the treatment site.

Moreover, as discussed above, suction may be applied through sheath 202. The particles and/or dust from the one or more objects may be removed through sheath 202, for example, through lumen 202C adjacent to the proximal portion (not shown) of irrigation device 206. Irrigation device 206 may have a smaller diameter or profile than shaft 208. In these aspects, larger particles and/or a larger amount of dust may be removed through lumen 202C of sheath 202, and/or there may be a reduced likelihood of the particles and/or dust forming one or more clogs in lumen 202C of sheath 202.

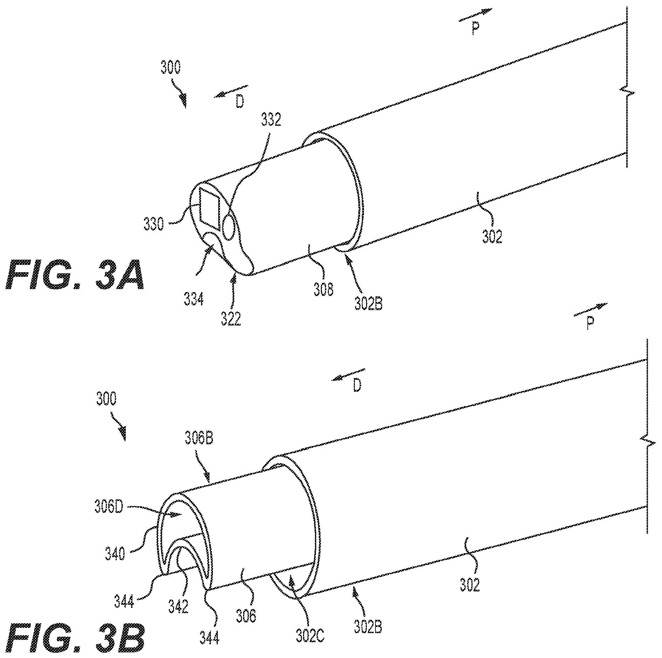

FIGs. 3A and 3B illustrate a medical system 300 (e.g., a distal portion of medical system 300) in various configurations. Where possible, corresponding reference numbers to medical system 100 (i.e., reference numbers including the same second and third digits) are used to refer to corresponding structure. As shown in FIG. 3A, a shaft 308 may be positioned within (e.g., radially within) a sheath 302, and a distal tip 322 of shaft 308 may extend distally beyond a distal end 302B of sheath 302. As mentioned above, distal tip 322 of shaft 308 may include one or more illumination devices 330, one or more imaging devices 332, and/or distal opening 334 of the working channel. As discussed, shaft 308 may be positioned within sheath 302 during the delivery and/or positioning of the distal end of medical system 300 at the treatment site. In these aspects, illumination device(s) 330 and/or imaging device(s) 332 may help the user visualize the treatment site and/or one or more lumens during the delivery to the treatment site. Furthermore, as mentioned above, one or more portions of shaft 308 may be deflectable (e.g., via one or more deflection levers 120, as shown in FIG. 1), which may help deliver and/or position sheath 302 at the treatment site. Although not shown, a laser fiber or other energy delivery device may be delivered to the treatment site (e.g., through the working channel or integrated in distal tip 322 of shaft 308). The laser fiber or other energy delivery device may be activated to break up the one or more objects at the treatment site. Moreover, illumination device(s) 330 and/or imaging device(s) 332 may help the user visualize the treatment site to help ensure that the one or more objects are sufficiently broken up. Furthermore, irrigation fluid may be delivered out of distal opening 334 of the working channel. Alternatively, suction may be applied through the working channel so that particles and/or dust from the one or more objects are removed through opening 334 of the working channel.

FIG. 3B illustrates the distal end of medical system 300 in another configuration. As shown, shaft 308 may be removed proximally from sheath 302. Additionally, an irrigation device 306 may be delivered through lumen 302C of sheath 302. A distal portion 306B of irrigation device 306 may be extended distally beyond distal end 302B of sheath 302. Irrigation fluid may be delivered through irrigation device 306 and out of the distal opening 306D of irrigation lumen. The irrigation fluid may help to flush one or more portions of the treatment site (e.g., one or more calyxes of a kidney). As discussed above, suction may be applied to the treatment site through sheath 302, for example, through lumen 302C. The suction may help to remove particles and/or dust formed when the laser fiber or other energy delivery device breaks up the one or more objects at the treatment site.

As shown in FIG. 3B, irrigation device 306 may include a crescent or "halfmoon" shape. For example, irrigation device 306 may include a rounded or semi-circular surface 340, for example, on an outer surface of irrigation device 306. Rounded surface 340 may abut an inner surface of lumen 302C of sheath 302. As shown, sheath 302 and lumen 302C may be generally cylindrical (e.g., with a circular cross-section). In these aspects, the interaction and/or abutment of rounded surface 340 with the inner surface of lumen 302C may help to bias irrigation device 306 to a position against one side of the inner surface of lumen 302C. In these aspects, as irrigation device 306 is extended distally or retracted proximally relative to sheath 302, rounded surface 340 may abut or slide against one side of the inner surface of lumen 302C. Irrigation device 306 may help to retain or maintain the position of irrigation device 306 within sheath 302, for example, with rounded surface 340 abutting the inner surface of lumen 302C of sheath 302. In this aspect, rounded surface 340 may form an exterior surface of irrigation device 306 that is substantially complementary to an interior surface of sheath 302, for example, a portion of the interior surface of lumen 302C. Irrigation device 306 may help to flush one or more portions of the treatment site (e.g., one or more calyxes of a kidney).

Irrigation device 306 also includes another surface, for example, an indented surface 342. Indented surface 342 may extend inward (e.g., toward rounded surface 340) from two transition points 344. In this aspect, particles and/or dust from the one or more objects that have been broken up may be removed through lumen 302C, for example, between indented surface 342 and the inner wall of lumen 302C.

Moreover, although not shown, irrigation device 306 may include one or more holes (e.g., rows of holes) positioned on one or more portions of distal portion 306B, for example, similar to the embodiments shown in FIGs. 1 and 2C. In these aspects, irrigation fluid may be delivered through the holes (and/or through distal opening 306D) to help flush the treatment site, such that particles and/or dust may then be removed through lumen 302C of sheath 302.

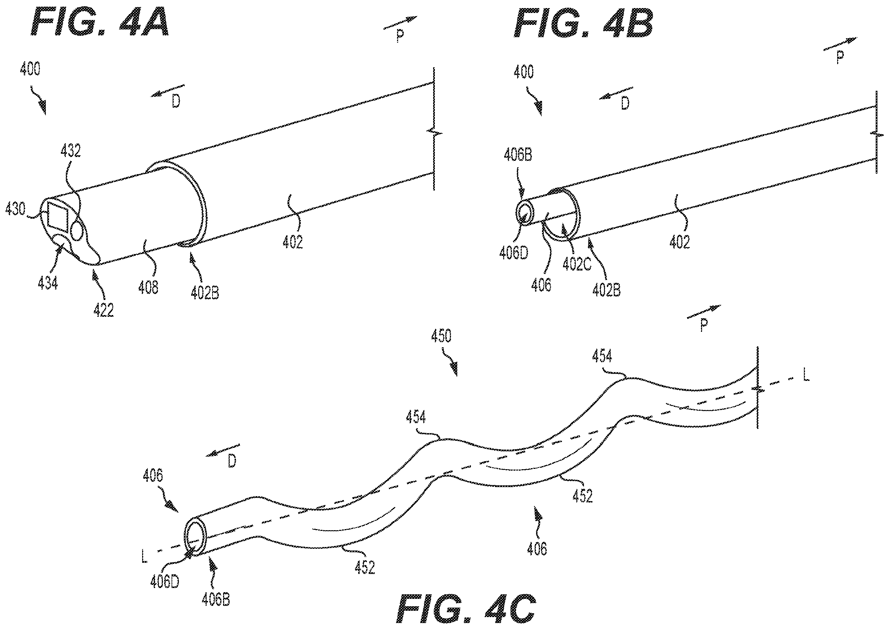

FIGs. 4A-4C illustrate a medical system 400 (e.g., a distal portion of medical system 400) in various configurations. Where possible, corresponding reference numbers to medical system 100 (i.e., reference numbers including the same second and third digits) are used to refer to corresponding structure. As shown in FIG. 4A, a shaft 408 may be positioned within (e.g., radially within) a sheath 402, and a distal tip 422 of shaft 408 may extend distally beyond a distal end 402B of sheath 402. As mentioned above, distal tip 422 of shaft 408 may include one or more illumination devices 430, one or more imaging devices 432, and/or distal opening 434 of the working channel.

As discussed above, shaft 408 may be positioned within sheath 402 during the delivery and/or positioning of the distal end of medical system 400 at the treatment site. In these aspects, illumination device(s) 430 and/or imaging device(s) 432 may help the user visualize the treatment site and/or one or more lumens during the delivery to the treatment site. Furthermore, as mentioned above, one or more portions of shaft 408 may be deflectable (e.g., via one or more deflection levers 120, as shown in FIG. 1), which may help deliver and/or position sheath 402 at the treatment site. Although not shown, a laser fiber or other energy delivery device may be delivered to the treatment site (e.g., through the working channel or integrated in distal tip 422 of shaft 408). The laser fiber or other energy delivery device may be activated to break up the one or more objects at the treatment site. Moreover, illumination device(s) 430 and/or imaging device(s) 432 may help the user visualize the treatment site to help ensure that the one or more objects are sufficiently broken up. Furthermore, irrigation fluid may be delivered out of distal opening 434 of the working channel. Alternatively, suction may be applied through the working channel so that particles and/or dust from the one or more objects are removed through opening 434 of the working channel.

FIG. 4B illustrates the distal end of medical system 400 in another configuration. As shown, shaft 408 may be removed proximally from sheath 402. Additionally, an irrigation device 406 may be delivered through lumen 402C of sheath 402. A distal portion 406B of irrigation device 406 may be extended distally beyond distal end 402B of sheath 402. Irrigation fluid may be delivered through irrigation device 406 and out of the distal opening 406D of an irrigation lumen. The irrigation fluid may help to flush one or more portions of the treatment site (e.g., one or more calyxes of a kidney). As discussed above, suction may be applied to the treatment site through sheath 402, for example, through lumen 402C. The suction may help to remove particles and/or dust formed when the laser fiber or other energy delivery device breaks up the one or more objects at the treatment site.

As shown in FIG. 4C, irrigation device 406 may include an at least partially helical portion 450 ("helical portion 450"). In some aspects, distal portion 406B, which terminates at distal opening 406D of irrigation lumen, may be generally cylindrical and straight. Helical portion 450 may extend proximally from distal portion 406B, as shown in FIG. 4C. Additionally, helical portion 450 may include one or more downward-extending portions 452 and one or more upward-extending portions 454. It is noted that "downward-extending" and "upward-extending" are used as relative terms to the orientation of FIG. 4C, and that downward-extending portions 452 and upward-extending portions 454 may be formed by helical portion extending helically (e.g., spirally). Irrigation device 406 may extend helically over helical portion 450, for example, relative to longitudinal axis L of irrigation device 406. In some aspects, downward-extending portion(s) 452 and upward-extending portion(s) 454 may abut respective portions of the inner wall of lumen 402C of sheath 402. In these aspects, downward-extending portion(s) 452 and upward-extending portion(s) 454 may help to retain or maintain the position of irrigation device 406 within sheath 402. In this aspect, downward-extending portion(s) 452 and upward-extending portion(s) 454 may form an exterior surface of irrigation device 406 that is substantially complementary to an interior surface of sheath 402, for example, a portion of the interior surface of lumen 402C.

Downward-extending portion(s) 452 and upward-extending portion(s) 454 of helical portion 450 may help to reduce the risk of blockages and/or help to break up blockages within lumen 402C of sheath 402. In these aspects, the outflow path for particles and/or dust being removed by the application of suction through lumen 402C may be at least partially tortuous, for example, around downward-extending portion(s) 452 and upward-extending portion(s) 454 of helical portion 450. Additionally, irrigation device 406 may be rotated, for example, clockwise or counterclockwise relative to longitudinal axis L. Rotating irrigation device 406, and thus helical portion 450, may help to break up and/or dislodge any particles or dust that accumulate within lumen 402C of sheath 402, for example, via a corkscrew or an auger-like movement. Irrigation device 406 may be distally extended and/or proximally retracted (whether alone or in combination with rotation), for example, along longitudinal axis L, which may help to break up and/or dislodge any particles or dust that accumulate within lumen 402C of sheath 402. The distal extension and/or proximal retraction may also help to position distal opening 406D of the irrigation lumen to help flush one or more portions of the treatment site (e.g., one or more calyxes of a kidney).

Moreover, although not shown, irrigation device 406 may include one or more holes (e.g., rows of holes) positioned on one or more portions of distal portion 406B, for example, similar to the embodiments shown in FIGs. 1 and 2C. In these aspects, irrigation fluid may be delivered through the holes (and/or through distal opening 406D) to help flush the treatment site, such that particles and/or dust may then be removed through lumen 402C of sheath 402.

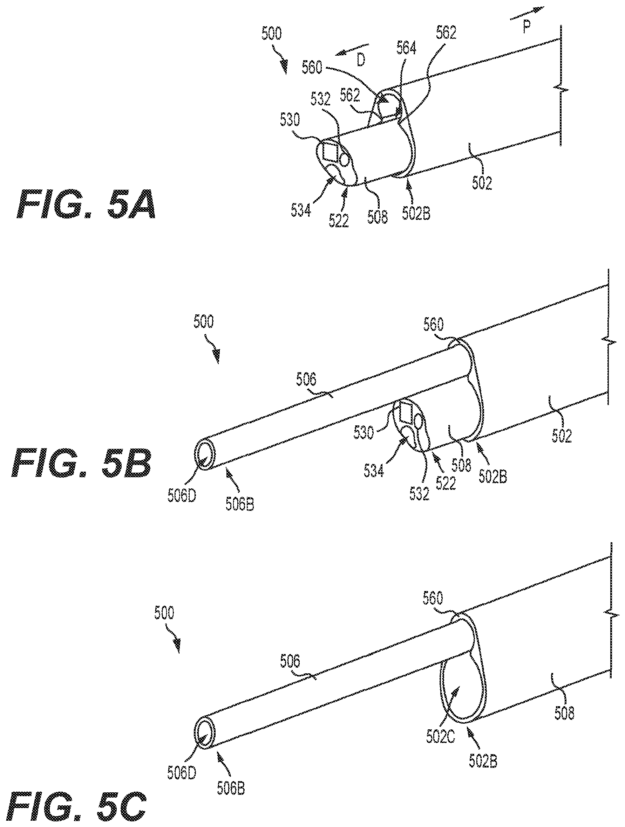

FIGs. 5A-5C illustrate a medical system 500 (e.g., a distal portion of medical system 500) in various configurations. Where possible, corresponding reference numbers to medical system 100 (i.e., reference numbers including the same second and third digits) are used to refer to corresponding structure. As shown in FIG. 5A, a shaft 508 may be positioned within (e.g., radially within) a sheath 502, and a distal tip 522 of shaft 508 may extend distally beyond a distal end 502B of sheath 502. As mentioned above, distal tip 522 of shaft 508 may include one or more illumination devices 530, one or more imaging devices 532, and/or distal opening 534 of the working channel. Shaft 508 may also include a deflectable portion. In these aspects, shaft 508 may help the user deliver and/or position medical system 500 to the treatment site. Furthermore, as discussed above, a laser fiber or other energy delivery device may be delivered through the working channel or otherwise be incorporated in shaft 508 to break up one or more objects at the treatment site.

As discussed above, sheath 502 includes a lumen 502C (FIG. 5C). In addition, sheath 502 includes a second or additional lumen 560. Additional lumen 560 may be smaller (e.g., having a smaller diameter) than lumen 502C. In these aspects, sheath 502 may include a teardrop-shaped or elliptical-shaped cross-section, for example, ovular with a narrower top and a wider bottom, as shown in FIGs. 5A-5C. Additionally, additional lumen 560 may be adjacent to lumen 502C. For example, sheath 502 may include one or more (e.g., two) inward extensions 562. In these aspects, sheath 502 may include a passage 564 between lumen 502C and additional lumen 560. Passage 564 may be narrower or smaller than a diameter of additional lumen 560. Alternatively, although not shown, inward extensions 562 may be joined to each other, thus separating lumen 502C and additional lumen 560. In any of these examples, irrigation device 506 may be at least partially retained or maintained within sheath 502, for example, within additional lumen 560. In this aspect, an exterior surface of irrigation device 506 may be substantially complementary to an interior surface of sheath 502, for example, a portion of the interior surface of additional lumen 560.

As shown in FIGs. 5B and 5C, an irrigation device 506 may be positioned within additional lumen 560. A distal portion 506B of irrigation device 506 may be extended distally beyond distal end 502B of sheath 502. Irrigation fluid may be delivered through irrigation device 506 and out of a distal opening 506D of an irrigation lumen. The irrigation fluid may help to flush one or more portions of the treatment site (e.g., one or more calyxes of a kidney). In some aspects, irrigation device 506 may be positioned at the treatment site (e.g., through additional lumen 560) with shaft 508 positioned in lumen 502C. As such, illumination device(s) 530 and/or imaging device(s) 532 may be activated and may aid the user in positioning irrigation device 506 at the treatment site. Moreover, shaft 508 may be deflected, which may also help the user position irrigation device 506 at the treatment site. Distal opening 534 of the working channel of shaft 508 may be used to apply suction to the treatment site, for example, to help remove particles and/or dust formed when the one or more objects are broken up.

As shown in FIG. 5C, shaft 508 may be removed proximally from lumen 502C. Moreover, as discussed above, suction may be applied to the treatment site through sheath 502, for example, through lumen 502C. The suction may help to remove particles and/or dust formed when the laser fiber or other energy delivery device breaks up the one or more objects at the treatment site. Furthermore, lumen 502C may form a large opening (e.g., larger than distal opening 534 of the working channel of shaft 508) to remove particles and/or dust, for example, at a greater rate, larger pieces (e.g., diameters) of particles, and/or larger amounts of particles and/or dust.

Moreover, although not shown, irrigation device 506 may include one or more holes (e.g., rows of holes) positioned on one or more portions of distal portion 506B, for example, similar to the embodiments shown in FIGs. 1 and 2C. In these aspects, irrigation fluid may be delivered through the holes (and/or through distal opening 506D) to help flush the treatment site, such that particles and/or dust may then be removed through lumen 502C of sheath 502 (or through distal opening 534 of the working channel of shaft 508).

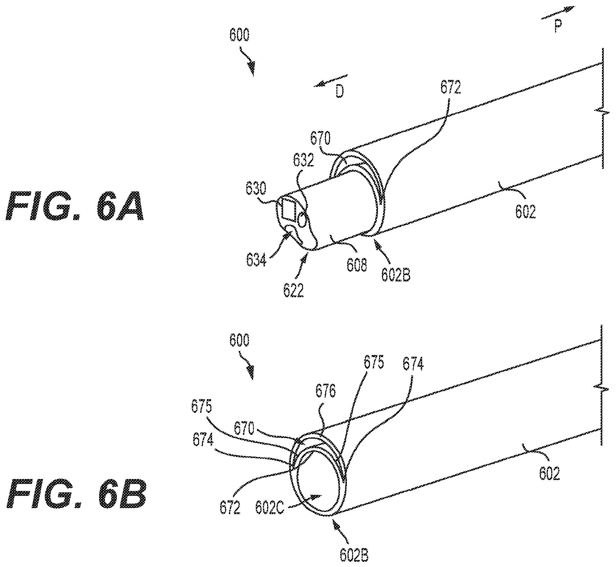

FIGs. 6A and 6B illustrate a medical system 600 (e.g., a distal portion of medical system 600) in various configurations. Where possible, corresponding reference numbers to medical system 100 (i.e., reference numbers including the same second and third digits) are used to refer to corresponding structure. As shown in FIG. 6A, a shaft 608 may be positioned within (e.g., radially within) a sheath 602, and a distal tip 622 of shaft 608 may extend distally beyond a distal portion 602B of sheath 602. As mentioned above, distal tip 622 of shaft 608 may include one or more illumination devices 630, one or more imaging devices 632, and/or distal opening 634 of the working channel. Shaft 608 may also include a deflectable portion. In these aspects, shaft 608 may help the user deliver and/or position medical system 600 to the treatment site. Furthermore, as discussed above, a laser fiber or other energy delivery device may be delivered through the working channel or otherwise be incorporated in shaft 608 to break up one or more objects at the treatment site.

As discussed above, sheath 602 includes a lumen 602C (FIG. 6B). Shaft 608 may be movably positioned within lumen 602C. In addition, sheath 602 includes a second or additional lumen 670. Additional lumen 670 may be crescent or "sliver moon" shaped. Additional lumen 670 may be smaller (e.g., having a smaller cross-sectional area) than lumen 602C. In these aspects, sheath 602 may include a circular cross-section. Lumen 602C may also have a circular cross-section, but may be positioned in a bottom or lower (e.g., relative to the orientation in FIGs. 6A and 6B) of sheath 602. Moreover, as shown in FIG. 6B, lumen 602C and additional lumen 670 may be separated by an inner wall 672. Inner wall 672 may be curved (e.g., with the same or similar curvature as an exterior of shaft 608), for example, with a smaller radius of curvature than the outer walls of sheath 602. Additional lumen 670 may be formed on the other side of inner wall 672 from lumen 602C. Additional lumen 670 may include two narrow portions 675 (FIG. 6B), for example, adjacent to transition points 674, and additional lumen 670 may also include a wider portion, for example, with a widest point 676. In some aspects, widest point 676 may be substantially equidistant in between transition points 674. For example, the wall of additional lumen 670 extending between transition points 674 (e.g., including widest point 676) may have a larger radius curvature than inner wall 672.

In these aspects, irrigation fluid may be delivered through additional lumen 670, in order to help flush one or more portions of the treatment site (e.g., one or more calyxes of a kidney). Alternatively or additionally, the irrigation fluid may help to clear an area of the treatment site distal to distal tip 622 of shaft 608 (e.g., to help visualize the treatment site with illumination device(s) 630 and imaging device(s) 632). Furthermore, as discussed above, suction may be applied via shaft 608, such that particles and/or dust from the one or more broken up objects at the treatment site may be removed proximally (e.g., through distal opening 634 of the working channel). Additionally, as shown in Fig. 6B, shaft 608 may be removed from lumen 602C, for example, after the one or more objects have been sufficiently broken up. In these aspects, suction may be applied to the treatment site directly via sheath 602 and lumen 602C. The suction may help to remove particles and/or dust formed when the laser fiber or other energy delivery device breaks up the one or more objects at the treatment site. Furthermore, lumen 602C may form a large opening (e.g., larger than distal opening 634 of the working channel of shaft 608) to remove particles and/or dust, for example, at a greater rate, larger pieces of particles, and/or larger amounts of particles and/or dust.

Moreover, additional lumen 670 may include one or more holes (e.g., rows of holes) positioned on one or more portions of sheath 602, for example, on a radial exterior of distal portion 602B of sheath 602. In these aspects, irrigation fluid may be delivered through the holes (and/or through the distal opening of additional lumen 670) to help flush the treatment site, such that particles and/or dust may then be removed through lumen 602C of sheath 602 (or through distal opening 634 of the working channel of shaft 608).

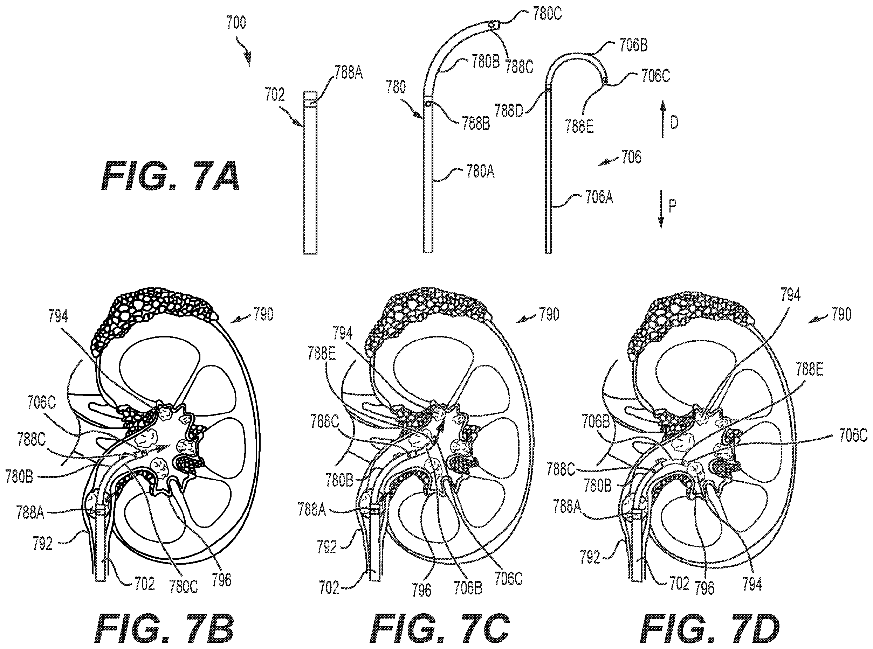

FIGs. 7A-7D illustrate a medical system 700 (e.g., a distal portion of medical system 700) in various configurations. Where possible, corresponding reference numbers to medical system 100 (i.e., reference numbers including the same second and third digits) are used to refer to corresponding structure. As shown in FIG. 7A, medical system 700 includes a sheath 702, a steering tube 780, and an inflow tube or an irrigation device 706, which may be concentrically arranged. For example, steering tube 780 may be positioned within and movable (e.g., longitudinally movable) relative to sheath 702. Irrigation device 706 may be positioned within and movable (e.g., longitudinally movably) relative to steering tube 780.

As discussed below, various aspects of medical system 700 may be delivered to and positioned at a treatment site (e.g., adjacent and/or within a kidney) without being guided under direct visualization (e.g., without an ureteroscope). In these aspects, various aspects of medical system 700 may be guided to the treatment site under ultrasound, fluoroscopic imaging, magnetic resonance imaging ("MRI"), or other indirect imaging modalities. For example, various aspects of medical system 700 may include one or more radiopaque markings or other indications 788A-788E (e.g., doping with barium sulfate) to aid in the indirect imaging modalities. The one or more markings or indications 788A-788E may be positioned at or adjacent to the distal ends of one or more of sheath 702, steering tube 780, and/or irrigation device 706. For example, one marking or indication 788A may be positioned adjacent to a distal end of sheath 702. One marking or indication 788B may be positioned on a first portion of steering tube 780, and another marking or indication 788C may be positioned on a second portion of steering tube 780, for example, adjacent to a distal end of steering tube 780. One marking or indication 788D may be positioned on a first portion of irrigation device 706, and another marking or indication 788E may be positioned on a second portion of irrigation device 706, for example, adjacent to a distal end of irrigation device 706. In these aspects, each of markings or indications 788A-788E may be different sizes, shapes, orientations, etc. Alternatively or additionally, the one or markings 788A-788E or indications may include gradients, e.g., extending proximally from respective distal ends, extending distally from proximal positions, or other arrangements on one or more of sheath 702, steering tube 780, and/or irrigation device 706.

Additionally, various aspects of medical system 700 may include a preformed shape, for example, formed of a shape-memory material. For example, as shown in FIG. 7A, sheath 702 may include a cylindrical shape, and may be more rigid than steering tube 780 and/or irrigation device 706. Steering tube 780 may include a preformed curvature. For example, a proximal portion 780A of steering tube 780 may be substantially straight. In an unconstrained configuration (e.g., extended from or separate from sheath 702), a distal portion 780B of steering tube 780 may include a curvature, for example, with a radius of curvature of approximately 45 degrees to approximately 135 degrees, for example, approximately 90 degrees. In these aspects, a distal tip 780C of steering tube 780 may face approximately 45 degrees to approximately 135 degrees, for example, approximately 90 degrees, relative to proximal portion 780A. Moreover, the degree to which distal portion 780B extends from sheath 702 may control the curvature of distal portion 780B. For example, when distal portion 780B is fully within sheath 702, distal tip 780C may be in line with proximal portion 780A of steering tube 780. As distal portion 780B is extended distally from sheath 702, distal portion 780B and distal tip 780C may gradually transition to the configuration shown in FIG. 7A. In some examples, distal portion 780B may be flexible and may be biased towards a curved configuration, such as one of the curved configuration shown in FIG. 7A.

Furthermore, irrigation device 706 may also include a preformed shape, for example, formed of a shape-memory material. For example, as shown in FIG. 7A, irrigation device 706 may include a preformed curvature. For example, a proximal portion 706A of irrigation device 706 may be substantially straight. When in an unconstrained configuration (e.g., extended from or separate from sheath 702 and/or steering tube 780) distal portion 706B of irrigation device 706 may include a curvature, for example, with a radius of curvature of approximately 90 degrees to approximately 270 degrees, for example, approximately 180 degrees. In these aspects, a distal tip 706C of irrigation device 706 may face 180 degrees relative to proximal portion 706A. Moreover, the degree to which distal portion 706B extends from steering tube 780 may control the curvature of distal portion 706B. For example, when distal portion 706B is fully within steering tube 780 and within sheath 702, distal tip 706C may be in line (e.g., linearly aligned) with proximal portion 706A of irrigation device 706. Then, as steering tube 780 and irrigation device 706 are extended distally from sheath 702, distal portion 780B and distal portion 706B may transition to the curvature of distal portion 780B of steering tube 780. As distal portion 706B of irrigation device 706 is extended distally from distal portion 780B of steering tube 780, distal portion 706B and distal tip 706C may gradually transition to the configuration shown in FIG. 7A.

In these aspects, irrigation device 706 may be less rigid that steering tube 780, and steering tube 780 may be less rigid than sheath 702. Accordingly, the user may control the relative positioning (e.g., distal extension/proximal retraction, angular or rotational arrangement, etc.) of one or more components of medical system 700 to help deliver and/or position one or more portions of medical system 700 (e.g., irrigation device 706) to one or more portions of the treatment site (e.g., a stone or a calyx in a kidney).

FIGs. 7B-7D illustrate the delivery of medical system 700 to a treatment site (e.g., to a subject's kidney). As shown in FIG. 7B, sheath 702 may be position proximal to a kidney 790, for example, within a ureter 792 (e.g., via insertion through the subject's urethra). Distal portion 780B of steering tube 780 may extend distally from sheath 702. With the pre-formed curvature of distal portion 780B of steering tube 780, distal portion 780B (e.g., distal tip 780C) may extend distally into the subject's kidney 790. As distal portion 780B is delivered to kidney 790, a majority of irrigation device 706 may be within steering tube 780, for example, with distal tip 706C extending from distal portion 780B.

As shown in FIG. 7C, once distal tip 780C of steering tube 780 is within kidney 790, irrigation device 706 may be extended distally from distal tip 780C, such that distal portion 706B extends from distal portion 780B of steering tube 780. With the preformed curvature of distal portion 706B of irrigation device 706, distal portion 706B (e.g., distal tip 706C) may extend distally toward a portion of kidney 790, for example, toward one or more stones 794 and/or toward one or more calyces 796. The extension or retraction of irrigation device 706 (i.e., distal portion 706B) relative to steering tube 780 and/or sheath 702 controls the deflection of distal portion 706B of irrigation device 706. As discussed above, irrigation fluid may be delivered to the treatment site through a lumen (e.g., shown by the arrows in FIGs. 7B and 7C) in irrigation device 706. Moreover, as discussed above, suction may be applied through a lumen (not shown) in one or more of sheath 702 and/or steering tube 780.

Furthermore, as shown in FIG. 7D, irrigation device 706 may be rotated, for example, rotated approximately 180 degrees relative to its longitudinal axis (see FIG. 4C). In this aspect, irrigation device 706 may be directed toward another portion of kidney 790, for example, toward one or more other stones 794 and/or toward one or more other calyces 796. Then, as discussed above, irrigation fluid may be delivered to the treatment site through a lumen (not shown) in irrigation device 706, and suction may be applied through a lumen (not shown) in one or more of sheath 702 and/or steering tube 780.

Although not shown, steering tube 780 may be rotated relative to sheath 702 to help position steering tube 780 and/or irrigation device 706. Additionally, a laser fiber or other energy delivery device may be delivered through the lumen in irrigation device 706 or through the lumen in steering tube 780. The laser fiber or other energy delivery device may be activated to help break up one or more objects (e.g., one or more stones 794). Moreover, although not shown, irrigation device 706 may include one or more holes, as discussed above. Furthermore, irrigation device 706 may include one or more helical portions, as discussed above.

The systems, devices, and methods discussed herein may help an operator to deliver medical treatment to a treatment site, for example, to break up and remove kidney stones or other hardened masses or objects within a subject. As discussed above, once the distal end of the medical system, including a sheath, is positioned at the treatment site, there is no need to remove or reposition the sheath to deliver fluid, deliver energy, apply suction, or otherwise treat the treatment site within the body cavity. In these aspects, a medical device (e.g., a shaft) and/or an irrigation device may be positioned relative to the sheath and/or the treatment site in order to deliver fluid, deliver energy, apply suction, or otherwise treat the treatment site.

Moreover, as discussed above, the medical device (e.g., the shaft) may be positioned within the sheath during the delivery of the sheath to the treatment site. In these aspects, one or more of illumination devices and/or imaging devices on a distal end of the shaft may be activated to provide visualization and help the user deliver the sheath and the shaft to the treatment site. Furthermore, a deflection lever on a handle of the medical device (FIG. 1) may be manipulated to help deflect one or more portions of the shaft, and also manipulate the sheath to help deliver the sheath and the shaft to the treatment site.

The inflow of irrigation fluid may help to clear the area around a distal tip of the shaft (e.g., to clear the area around one or more of illumination devices and/or imaging devices) to help the user visualize the body lumen(s) and/or the treatment site. The inflow of irrigation fluid may help to flush one or more portions of the treatment site (e.g., one or more calyxes of a kidney). Furthermore, as discussed above, suction may be applied through the sheath, such that particles and/or dust may be removed proximally from the treatment site via a lumen of the sheath and around the shaft of the medical device.

Various aspects discussed herein may help to reduce the likelihood of one or more clogs or blockages forming in the sheath. For example, as discussed above, the medical device may be removed from the sheath to provide a larger opening (e.g., larger cross-sectional opening) for suction to be applied to remove fluid, along with one or more particles and/or dust. Additionally, as discussed above with respect to FIGs. 3B, 4B, and 4C, the irrigation device may be at least partially retained or maintained in a position relative to the sheath. Moreover, as discussed with respect to FIGs. 4B and 4C, the irrigation device may include a helical portion, which may be movable (e.g., extended distally, retracted proximally, rotated clockwise, and/or rotated counterclockwise), which may help to break up one or more clogs of blockages in the sheath. In these aspects, the medical systems, devices, and methods discussed herein may be used to break up and remove one or more particles or dust from the one or more objects without the need for a basket or other retrieval device. Additionally, irrigation fluid may be delivered simultaneously and/or alternatingly with the application of suction, for example, to help flush one or more portions of the treatment site (e.g., one or more calyxes in a kidney) and/or to maintain an appropriate or safe pressure at the treatment site.

While principles of this disclosure are described herein with reference to illustrative examples for particular applications, it should be understood that the disclosure is not limited thereto. Those having ordinary skill in the art and access to the teachings provided herein will recognize additional modifications, applications, and embodiments all fall within the scope of the features described herein. Accordingly, the claimed features are not to be considered as limited by the foregoing description.

## Claims

1. A medical system (100), comprising:
a sheath (102) with at least one lumen (102C), wherein the at least one lumen (102C) is configured to be coupled to a suction source;
an irrigation device (106), wherein the irrigation device (106) includes at least one irrigation lumen configured to be coupled to an irrigation source; and
a medical device (104), wherein the medical device (104) includes a handle (110) and a shaft (108), wherein the shaft (108) includes a working channel with a distal opening at a distal end of the shaft (108), wherein the distal end of the shaft (108) further includes one or more illumination devices and one or more visualization devices,
wherein the medical device (104) is configured to be positioned within the sheath (102) during delivery of the sheath (102) to a treatment site and during an application of energy to one or more objects to break up the one or more objects to form one or more particles or dust, and
wherein the irrigation device (106) is configured to be positioned within the sheath (102) and deliver irrigation fluid while suction is applied to the treatment site through the sheath (102) during a removal of one or more particles or dust from the treatment site, and
wherein a distal portion of the shaft (108) of the medical device (104) includes an articulation section (124), and wherein the handle (110) of the medical device (104) includes a deflection lever (120) that is configured to be manipulated to control a position of the articulation section (124) of the shaft (108).

2. The medical system (100) of claim 1, further comprising a laser fiber (236) or another energy delivered device.

3. The medical system (100) of claim 2, wherein the laser fiber (230) or the another energy delivery device is delivered through the working channel of the medical device (104).

4. The medical system (200) of claim 2, wherein the laser fiber (230) or the another energy delivery device is coupled to or integrated within the shaft (208) of the medical device (204).

5. The medical system (100) of any preceding claim, wherein the irrigation device (106) includes a closed distal end (106C) and a plurality of holes (112) arranged on a distal portion (106B) of the irrigation device (106).

6. The medical system (100) of claim 5, wherein the plurality of holes (112) are arranged in at least one row of holes.

7. The medical system (100) of claim 6, wherein the at least one row of holes (112) includes at least two rows of holes (112) arranged on opposing sides of the distal portion (106B) of the irrigation device (106), wherein the plurality of holes (112) are formed via laser cutting.

8. The medical system (300) of any preceding claim, wherein the irrigation device (306) includes a crescent cross-sectional shape with an open distal end (306D).

9. The medical system (400) of claim 1, wherein the irrigation device (406) includes at least one helical section (450).

10. The medical system (500) of claim 1, wherein the sheath (502) includes an additional lumen (560), wherein the additional lumen (560) is at least partially separated from the lumen (502C) of the sheath (502).

11. The medical system (500) of claim 10, wherein the additional lumen (560) is configured to movably receive the irrigation device (506) while the shaft (508) of the medical device (504) is positioned within the lumen (502C) of the sheath (502).

12. The medical system (600) of claim 10 or claim 11, wherein the additional lumen (670) includes a sliver shape formed by a first wall (672) with a first radius of curvature and a second wall with a second radius of curvature that is greater than the first radius of curvature.

13. The medical system (100) of any preceding claim, wherein the handle (110) is coupled to an umbilicus (126) configured to couple the handle (110) to one or more of an irrigation source, a suction source, a display, or a power source.

14. The medical system (100) of any preceding claim, wherein the medical device (104) is an ureteroscope, wherein the treatment site is a kidney (790), and wherein the one or more objects are kidney stones (794).

## Patentansprüche

1. Medizinisches System (100), umfassend:
eine Hülle (102) mit mindestens einem Lumen (102C), wobei das mindestens eine Lumen (102C) eingerichtet ist, an eine Saugquelle angeschlossen zu werden;
eine Spülvorrichtung (106), wobei die Spülvorrichtung (106) mindestens ein Spüllumen umfasst, das eingerichtet ist, an eine Spülquelle angeschlossen zu werden; und
eine medizinische Vorrichtung (104), wobei die medizinische Vorrichtung (104) einen Griff (110) und einen Schaft (108) umfasst, wobei der Schaft (108) einen Arbeitskanal mit einer distalen Öffnung an einem distalen Ende des Schafts (108) umfasst, wobei das distale Ende des Schafts (108) ferner eine oder mehrere Beleuchtungsvorrichtungen und eine oder mehrere Visualisierungsvorrichtungen umfasst,
wobei die medizinische Vorrichtung (104) eingerichtet ist, während des Hinführens der Hülle (102) zu einer Behandlungsstelle und während der Anwendung von Energie auf ein oder mehrere Objekte in der Hülle (102) positioniert ist, um das eine oder die mehreren Objekte aufzubrechen und so ein oder mehrere Partikel oder Staub zu bilden, und
wobei die Spülvorrichtung (106) eingerichtet ist, innerhalb der Hülle (102) positioniert zu werden und Spülflüssigkeit abzugeben, während ein Sog auf die Behandlungsstelle ausgeübt wird während einer Entfernung eines oder mehrerer Partikel oder Staubpartikel von der Behandlungsstelle durch die Hülle (102), und
wobei ein distaler Teil des Schafts (108) der medizinischen Vorrichtung (104) einen Artikulationsabschnitt (124) aufweist und wobei der Griff (110) der medizinischen Vorrichtung (104) einen Umlenkhebel (120) aufweist, der eingerichtet ist, betätigt zu werden, um eine Position des Artikulationsabschnitts (124) des Schafts (108) zu steuern.

2. Medizinisches System (100) gemäß Anspruch 1, das ferner eine Laserfaser (236) oder eine andere Vorrichtung zur Energieabgabe umfasst.

3. Medizinisches System (100) gemäß Anspruch 2, wobei die Laserfaser (230) oder die andere Energieliefervorrichtung durch den Arbeitskanal der medizinischen Vorrichtung (104) geführt ist.

4. Medizinisches System (200) gemäß Anspruch 2, wobei die Laserfaser (230) oder eine andere Energieliefervorrichtung an den Schaft (208) der medizinischen Vorrichtung (204) gekoppelt oder in diesen integriert ist.

5. Medizinische System (100) gemäß einem der vorhergehenden Ansprüche, wobei die Spülvorrichtung (106) ein geschlossenes distales Ende (106C) und eine Vielzahl von Löchern (112) aufweist, die an einem distalen Teil (106B) der Spülvorrichtung (106) angeordnet sind.

6. Medizinisches System (100) gemäß Anspruch 5, wobei die Vielzahl von Löchern (112) in mindestens einer Lochreihe angeordnet ist.

7. Medizinisches System (100) gemäß Anspruch 6, wobei die mindestens eine Lochreihe (112) mindestens zwei Lochreihen (112) umfasst, die an gegenüberliegenden Seiten des distalen Teils (106B) der Spülvorrichtung (106) angeordnet sind, wobei die Vielzahl von Löchern (112) durch Laserschneiden hergestellt ist.

8. Medizinisches System (300) gemäß einem der vorhergehenden Ansprüche, wobei die Spülvorrichtung (306) einen halbmondförmigen Querschnitt mit einem offenen distalen Ende (306D) aufweist.

9. Medizinisches System (400) gemäß Anspruch 1, wobei die Spülvorrichtung (406) mindestens einen spiralförmigen Abschnitt (450) umfasst.

10. Medizinisches System (500) gemäß Anspruch 1, wobei die Hülle (502) ein zusätzliches Lumen (560) aufweist, wobei das zusätzliche Lumen (560) zumindest teilweise vom Lumen (502C) der Hülle (502) getrennt ist.

11. Medizinisches System (500) gemäß Anspruch 10, wobei das zusätzliche Lumen (560) eingerichtet ist, die Spülvorrichtung (506) beweglich aufzunehmen, während sich der Schaft (508) der medizinischen Vorrichtung (504) innerhalb des Lumens (502C) der Hülle (502) befindet.

12. Medizinisches System (600) gemäß Anspruch 10 oder Anspruch 11, wobei das zusätzliche Lumen (670) eine schmalstreifenförmige Gestalt aufweist, die durch eine erste Wand (672) mit einem ersten Krümmungsradius und eine zweite Wand mit einem zweiten Krümmungsradius gebildet wird, der größer ist als der erste Krümmungsradius.

13. Medizinisches System (100) gemäß einem der vorhergehenden Ansprüche, wobei der Griff (110) mit einem Anschluss (126) verbunden ist, der eingerichtet ist, den Griff (110) mit einer oder mehreren der folgenden Komponenten zu verbinden: einer Spülquelle, einer Absaugquelle, einer Anzeige oder einer Energiequelle.

14. Medizinisches System (100) gemäß einem der vorhergehenden Ansprüche, wobei die medizinische Vorrichtung (104) ein Ureteroskop ist, wobei die Behandlungsstelle eine Niere (790) ist und wobei es sich bei dem einen oder den mehreren Objekten um Nierensteine (794) handelt.

## Revendications

1. Système médical (100), comprenant :
une gaine (102) qui comporte au moins une lumière (102C), dans lequel l'au moins une lumière (102C) est configurée pour être couplée à une source d'aspiration ;
un dispositif d'irrigation (106), dans lequel le dispositif d'irrigation (106) comprend au moins une lumière d'irrigation configurée pour être couplée à une source d'irrigation ; et
un dispositif médical (104), dans lequel le dispositif médical (104) comprend une poignée (110) et un arbre (108), dans lequel l'arbre (108) comprend un canal de travail qui présente une ouverture distale au niveau d'une extrémité distale de l'arbre (108), et dans lequel l'extrémité distale de l'arbre (108) comprend en outre un ou plusieurs dispositifs d'éclairage et un ou plusieurs dispositifs de visualisation, dans lequel :
le dispositif médical (104) est configuré pour être positionné à l'intérieur de la gaine (102) pendant l'acheminement de la gaine (102) jusqu'à un site de traitement et pendant l'application d'énergie à un ou plusieurs objets afin de fragmenter les un ou plusieurs objets pour former une ou plusieurs particules ou de la poussière, et dans lequel :
le dispositif d'irrigation (106) est configuré pour être positionné à l'intérieur de la gaine (102) et pour délivrer un fluide d'irrigation tandis qu'une aspiration est appliquée au niveau du site de traitement à travers la gaine (102) lors du retrait d'une ou de plusieurs particules ou de la poussière hors du site de traitement, et dans lequel :
une partie distale de l'arbre (108) du dispositif médical (104) comprend une section d'articulation (124), et dans lequel la poignée (110) du dispositif médical (104) comprend un levier de déflexion (120) qui est configuré pour être manipulé afin de commander une position de la section d'articulation (124) de l'arbre (108).

2. Système médical (100) selon la revendication 1, comprenant en outre une fibre laser (236) ou un autre dispositif de délivrance d'énergie.

3. Système médical (100) selon la revendication 2, dans lequel la fibre laser (230) ou l'autre dispositif de délivrance d'énergie est acheminé(e) à travers le canal de travail du dispositif médical (104).

4. Système médical (200) selon la revendication 2, dans lequel la fibre laser (230) ou l'autre dispositif de délivrance d'énergie est couplé(e) à l'arbre (208) du dispositif médical (204) ou est intégré(e) à l'intérieur de celui-ci.

5. Système médical (100) selon l'une quelconque des revendications précédentes, dans lequel le dispositif d'irrigation (106) comprend une extrémité distale fermée (106C) et une pluralité de trous (112) agencés sur une partie distale (106B) du dispositif d'irrigation (106).

6. Système médical (100) selon la revendication 5, dans lequel les trous de la pluralité de trous (112) sont agencés selon au moins une rangée de trous.

7. Système médical (100) selon la revendication 6, dans lequel l'au moins une rangée de trous (112) comprend au moins deux rangées de trous (112) agencées sur des côtés opposés de la partie distale (106B) du dispositif d'irrigation (106), et dans lequel les trous de la pluralité de trous (112) sont formés par découpe au laser.

8. Système médical (300) selon l'une quelconque des revendications précédentes, dans lequel le dispositif d'irrigation (306) présente une forme en coupe transversale en croissant qui est munie d'une extrémité distale ouverte (306D).

9. Système médical (400) selon la revendication 1, dans lequel le dispositif d'irrigation (406) comprend au moins une section hélicoïdale (450).

10. Système médical (500) selon la revendication 1, dans lequel la gaine (502) comprend une lumière supplémentaire (560), et dans lequel la lumière supplémentaire (560) est au moins partiellement séparée de la lumière (502C) de la gaine (502).

11. Système médical (500) selon la revendication 10, dans lequel la lumière supplémentaire (560) est configurée pour recevoir de manière mobile et amovible le dispositif d'irrigation (506) tandis que l'arbre (508) du dispositif médical (504) est positionné à l'intérieur de la lumière (502C) de la gaine (502).

12. Système médical (600) selon la revendication 10 ou la revendication 11, dans lequel la lumière supplémentaire (670) présente une forme de lamelle effilée qui est définie par une première paroi (672) qui présente un premier rayon de courbure et par une seconde paroi qui présente un second rayon de courbure plus grand que le premier rayon de courbure.

13. Système médical (100) selon l'une quelconque des revendications précédentes, dans lequel la poignée (110) est couplée à un cordon (126) configuré pour coupler la poignée (110) à un ou plusieurs moyens parmi une source d'irrigation, une source d'aspiration, un écran d'affichage et une source d'alimentation.

14. Système médical (100) selon l'une quelconque des revendications précédentes, dans lequel le dispositif médical (104) est un urétéroscope, dans lequel le site de traitement est un rein (790), et dans lequel lesdits un ou plusieurs objets sont des calculs rénaux (794).
